# EUROPEAN PATENT APPLICATION

(11) **EP 4 529 763 A1**
(43) Date of publication of application: **02.04.2025**
(21) Application number: 24189933.5
(22) Date of filing: 22.07.2024
(51) Int. Cl.: A01M 7/00

(54) **SPRAYER VEHICLES, LIQUID DISTRIBUTION SYSTEMS FOR SPRAYER VEHICLES COMPRISING A WATER QUALITY SENSOR, AND RELATED METHODS**

(30) Priority: 27.09.2023 US 202363585626 P
(71) Applicant: Agco Corporation, Duluth, Georgia 30096 (US)
(72) Inventor: BAK, Justin Thomas, Jackson, 56143 (US); SNYDER, Todd Brandon, Jackson, 56143 (US)
(74) Representative: AGCO Intellectual Property Department

(57) **Abstract**

A liquid distribution system for a sprayer vehicle includes a product tank configured to contain a liquid and having a fluid inlet and a fluid outlet; a pump in fluid communication with the product tank; a plurality of nozzles carried by a boom; a recirculation line connecting the fluid outlet, the pump, at least some of the plurality of nozzles, and the fluid inlet; a bypass line connected at an upstream connection and a downstream connection to the recirculation line; and at least one water quality sensor configured to detect a water quality property of liquid flowing through the bypass line. Related vehicles and methods are also disclosed.

## Description

### FIELD

Embodiments of the present disclosure relate generally to liquid distribution systems for a sprayer vehicle, and more particularly to liquid distribution system having one or more water quality sensors.

### BACKGROUND

High crop yields of modern agribusiness typically require application of fertilizers, pesticides, fungicides, and/or herbicides. Dispersing these chemicals onto high-acreage fields requires specialized machines mounted on or towed by a vehicle. An example of such a machine is the self-propelled sprayer.

A common design for a self-propelled crop sprayer includes a chassis with a tank, boom arms, and nozzles connected to the boom arms. The tank contains liquid product such as fertilizers, pesticides, fungicides, and/or herbicides. Boom arms extend outward from the sides of the chassis. Boom plumbing contains supply lines and nozzles spaced apart along the length of the boom arms at a spacing distance corresponding to the spray pattern of the nozzles. In operation, as the crop sprayer crosses the field, liquid is pumped from the tank through the supply lines along the boom arms, and out through the nozzles. This allows the self-propelled sprayer to distribute the liquid along a relatively wide path. The length of conventional boom arms may vary from, for example, 6 meters (18 feet) up to 46 meters (150 feet), but shorter or longer booms are possible. The boom arms typically swing in for on-road transport and out for field-spraying operations.

Conventionally, the nozzles are connected in series such that the product flows through a pipe and/or hose from one nozzle to another. Booms have been of the "wet boom" type, and where the boom comprises a frame member with a pipe mounted thereon, where the liquid passes through the pipe into nozzles mounted on the pipe and liquidly connected thereto, or a "dry boom" type, where the nozzles are mounted to the frame member and liquid passes to the nozzles through a hose which is connected between the nozzles. The nozzles are attached to the pipe or frame with brackets at desired intervals along the boom arm.

When a sprayer is first used to dispense a particular product, the product may be flushed through the pipe or hose to each nozzle to fill entire plumbing system with the product. This may help to remove air bubbles and ensure that any prior products are purged from the system. To avoid uneven distribution of the product, this flushing and purging process may be performed before the sprayer enters the planted area of the field. This process may dispense several gallons of product out of the boom to ensure that all of the air is out of the boom and that liquid product can consistently and evenly be dispensed from each of the nozzles.

### BRIEF SUMMARY

In some embodiments, a liquid distribution system for a sprayer vehicle includes a product tank configured to contain a liquid and having a fluid inlet and a fluid outlet; a pump in fluid communication with the product tank; a plurality of nozzles carried by a boom; a recirculation line connecting the fluid outlet, the pump, at least some of the plurality of nozzles, and the fluid inlet; a bypass line connected at an upstream connection and a downstream connection to the recirculation line; and at least one water quality sensor configured to detect a water quality property of liquid flowing through the bypass line.

The liquid distribution system may optionally include at least one valve configured to control flow through the bypass line.

The at least one water quality sensor may include, without limitation, a pH sensor, a turbidity sensor, a dissolved oxygen sensor, a salinity sensor, an ammonia sensor, a chlorine sensor, a nitrate sensor, a sulfite sensor, and/or a total organic carbon sensor. The water quality sensor may be configured to report the water quality property to an operator of the sprayer vehicle.

The liquid distribution system may include a rinse tank configured to contain rinse water separate from the liquid in the product tank and to selectively provide rinse water to the recirculation line.

A sprayer vehicle includes the liquid distribution system as described. The sprayer vehicle may include a self-propelled chassis carrying the liquid distribution system, or a towed chassis carrying the liquid distribution system.

A method of operating a sprayer vehicle includes spraying a first liquid from a fluid outlet of a product tank of the sprayer vehicle through a plurality of nozzles carried by a boom; providing a rinse fluid in the product tank; passing the rinse fluid from the fluid outlet of the product tank, past the plurality of nozzles, and through a recirculation line to a fluid inlet of the product tank or to a rinse collection container external to the sprayer vehicle; diverting at least a portion of the rinse fluid through a bypass line; and measuring a water quality property of the rinse fluid in the bypass line.

The method optionally includes draining the product tank before providing the rinse fluid to the product tank.

The method optionally includes purging the rinse fluid from the sprayer vehicle. The rinse fluid may be collected in a rinse collection container.

The water quality property measured may include, without limitation, pH, turbidity, dissolved oxygen, salinity, ammonia concentration, chloride concentration, nitrate concentration, sulfite concentration, total organic carbon, concentration of an element, and/or concentration of any ion.

If the measured water quality property is greater than a preselected threshold, additional rinse fluid may be provided to the product tank for additional rinsing.

### BRIEF DESCRIPTION OF THE DRAWINGS

While the specification concludes with claims particularly pointing out and distinctly claiming what are regarded as embodiments of the present disclosure, various features and advantages may be more readily ascertained from the following description of example embodiments when read in conjunction with the accompanying drawings, in which:
FIG. 1 is a simplified perspective view illustrating an agricultural vehicle in the form of a self-propelled sprayer vehicle;
FIG. 2 illustrates a portion of the agricultural vehicle of FIG. 1 including a boom and part of a liquid distribution system;
FIG. 3 is a simplified schematic showing flow of fluid in a liquid distribution system of the agricultural vehicle of FIG. 1;
FIG. 4 is a simplified view of a bypass line of the liquid distribution system of FIG. 3;
FIG. 5 is a simplified schematic showing flow of fluid in another liquid distribution system;
FIG. 6 is a simplified top view of another agricultural vehicle, in the form of a sprayer vehicle towed by a tractor;
FIG. 7 is a simplified flow chart illustrating a method of operating a sprayer vehicle; and
FIG. 8 is a simplified flow chart illustrating another method of operating a sprayer vehicle.

### DETAILED DESCRIPTION

The illustrations presented herein are not actual views of any agricultural machine or portion thereof, but are merely idealized representations to describe example embodiments of the present disclosure. Additionally, elements common between figures may retain the same numerical designation.

The following description provides specific details of embodiments. However, a person of ordinary skill in the art will understand that the embodiments of the disclosure may be practiced without employing many such specific details. Indeed, the embodiments of the disclosure may be practiced in conjunction with conventional techniques employed in the industry. In addition, the description provided below does not include all elements to form a complete structure or assembly. Only those process acts and structures necessary to understand the embodiments of the disclosure are described in detail below. Additional conventional acts and structures may be used. The drawings accompanying the application are for illustrative purposes only, and are thus not drawn to scale.

As used herein, the terms "comprising," "including," "containing," "characterized by," and grammatical equivalents thereof are inclusive or open-ended terms that do not exclude additional, unrecited elements or method steps, but also include the more restrictive terms "consisting of" and "consisting essentially of" and grammatical equivalents thereof.

As used herein, the term "may" with respect to a material, structure, feature, or method act indicates that such is contemplated for use in implementation of an embodiment of the disclosure, and such term is used in preference to the more restrictive term "is" so as to avoid any implication that other, compatible materials, structures, features, and methods usable in combination therewith should or must be excluded.

As used herein, the term "configured" refers to a size, shape, material composition, and arrangement of one or more of at least one structure and at least one apparatus facilitating operation of one or more of the structure and the apparatus in a predetermined way.

As used herein, the singular forms following "a," "an," and "the" are intended to include the plural forms as well, unless the context clearly indicates otherwise.

As used herein, the term "and/or" includes any and all combinations of one or more of the associated listed items.

As used herein, spatially relative terms, such as "beneath," "below," "lower," "bottom," "above," "upper," "top," "front," "rear," "left," "right," and the like, may be used for ease of description to describe one element's or feature's relationship to another element(s) or feature(s) as illustrated in the figures. Unless otherwise specified, the spatially relative terms are intended to encompass different orientations of the materials in addition to the orientation depicted in the figures.

As used herein, the term "substantially" in reference to a given parameter, property, or condition means and includes to a degree that one of ordinary skill in the art would understand that the given parameter, property, or condition is met with a degree of variance, such as within acceptable manufacturing tolerances. By way of example, depending on the particular parameter, property, or condition that is substantially met, the parameter, property, or condition may be at least 90.0% met, at least 95.0% met, at least 99.0% met, or even at least 99.9% met.

As used throughout, ranges are used as shorthand for describing each and every value that is within the range. Any value within the range can be selected as the terminus of the range.

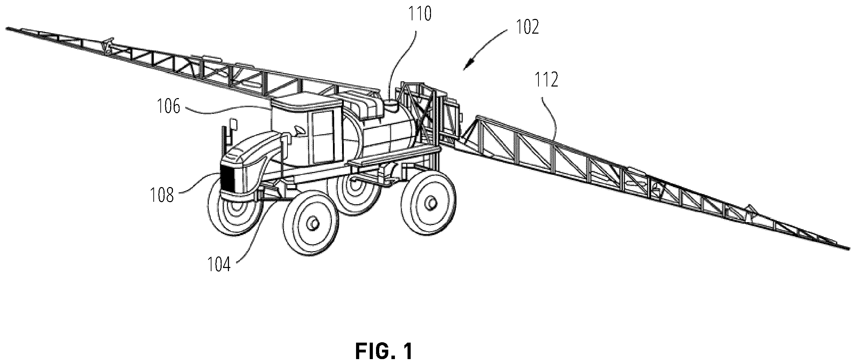

FIG. 1 shows a sprayer vehicle 102 used to deliver chemicals to agricultural crops in a field. A sprayer vehicle may also be referred to in the art as a crop sprayer. The sprayer vehicle 102 includes a self-propelled chassis 104 and an operator cab 106 mounted on the chassis 104. The operator cab 106 may house controls for the sprayer vehicle 102. An engine 108 may be mounted on a forward portion of the chassis 104 in front of operator cab 106 or may be mounted on a rearward portion of the chassis 104 behind the operator cab 106. The engine 108 may be commercially available from a variety of sources and may include, for example, a diesel engine or a gasoline-powered internal combustion engine, a battery-powered electric motor, *etc.* The engine 108 provides energy to propel the sprayer vehicle 102 through a field on wheels or tracks, and may also provide energy to spray liquids from the sprayer vehicle 102.

The sprayer vehicle 102 further includes a product tank 110 to store a liquid to be sprayed on the field. The liquid may include chemicals, such as but not limited to, herbicides, pesticides, fungicides, and/or fertilizers. The product tank 110 may be mounted on the chassis 104, either in front of or behind the operator cab 106. The sprayer vehicle 102 may include more than one product tank 110 to store different chemicals to be sprayed on the field. The stored chemicals may be dispersed by the sprayer vehicle 102 one at a time, or different chemicals may be mixed and dispersed together in a variety of mixtures.

A boom 112 on the sprayer vehicle 102 is used to distribute the liquid from the product tank 110 over a wide swath as the sprayer vehicle 102 is driven through the field. The boom 112 may include two or more portions that can fold for transport on public roadways, and unfold for field operations (*i*.*e*., to the position shown in FIG. 1).

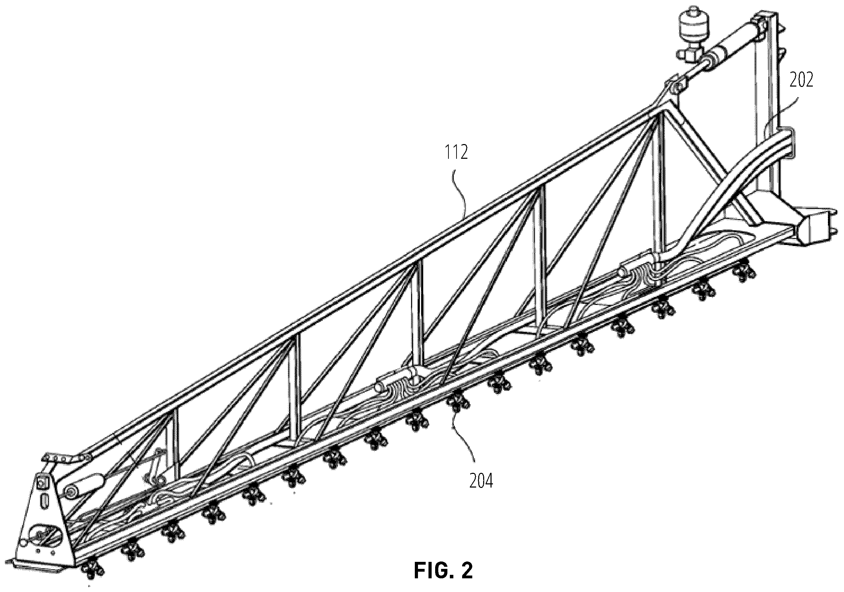

FIG. 2 is a simplified perspective view of a portion of the boom 112. Liquid is conveyed from the product tank 110 (FIG. 1) by a liquid distribution system 202 to a plurality of nozzles 204 carried by and spaced along the boom 112. The liquid distribution system 202, which may be mounted on the boom 112, includes at least one supply line and, optionally, a recirculation line connected to the product tank 110. Recirculation of liquid to the product tank 110 may be useful for purging lines when changing products without wasting product onto the ground or damaging crops with excess product. In some embodiments, the liquid distribution system 202 can be purged at a shop or mixing plant, where liquid can be captured, such as on a catch pad or other container.

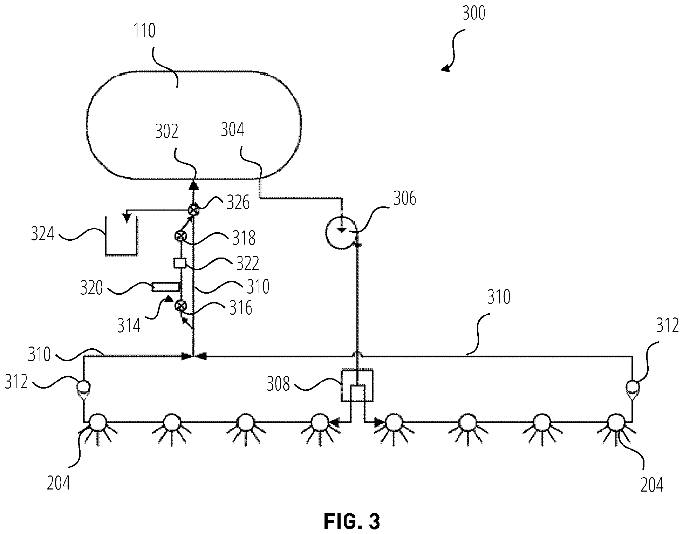

FIG. 3 is a simplified schematic view of a liquid distribution system 300, such as the liquid distribution system 202 carried by the sprayer vehicle 102. The liquid distribution system 300 includes the product tank 110 configured to contain a liquid to be applied to a field (or over any other area, such as a roadway, runway, parking lot, *etc.*)*.* The product tank 110 has a fluid inlet 302 and a fluid outlet 304, such that the fluid in the product tank 110 can be sprayed through the nozzles 204 carried by the boom 112 (FIG. 2) and, optionally, recirculated through the liquid distribution system 300 back to the product tank 110. The product tank 110 may include one or more mixing devices, such as a sparge tube, an agitator, *etc.,* to promote uniform mixing of the liquid within the product tank 110.

As shown, a pump 306 is in fluid communication with the product tank 110. The nozzles 204 are configured to receive liquid from the product tank 110 via the pump 306, and optionally, a flow splitter 308 (*e.g.,* to split flow into two or more portions to supply different sections of the boom 112). The pump 306 may be connected to the fluid outlet 304 of the product tank 110 by appropriate plumbing lines, or may be within the product tank 110. The product tank 110 is generally filled with fresh water from an external source (*e*.*g*., a well, a pond, a canal, a municipal supply, *etc.*)*.* One or more chemicals (*e.g.,* a nutrient, a fungicide, an herbicide, an insecticide, *etc.*) can be mixed with the water in the product tank 110, and may be mixed before, during, or after the product tank 110 is filled.

As shown, the pump 306 provides liquid through the flow splitter 308 to the nozzles 204. The liquid distribution system 300 includes a recirculation line 310 connecting the nozzles 204 to the fluid inlet 302 of the product tank 110. When the nozzles 204 are not spraying (and in some embodiments, even when the nozzles 204 are spraying but are spraying less volume than the pump 306 delivers), the liquid or a portion thereof passes through the recirculation line 310 and returns to the product tank 110 through the fluid inlet 302. One or more check valves 312 may ensure that liquid does not flow backward through the recirculation line 310 from the fluid inlet 302 of the product tank 110 to the nozzles 204.

The recirculation line 310 may be used to prime the liquid distribution system 300 to apply a different liquid, to clean out the liquid distribution system 300 after use, and/or to promote uniform mixing throughout a liquid distribution system 300.

The liquid distribution system 300 may recirculate fluid while the nozzles 204 are spraying liquid onto the field, or while the nozzles 204 are not spraying (depending on the pressure at the nozzles 204). That is, the nozzles 204 may spray liquid only when pressure at the nozzles 204 is above a threshold. When flushing or cleaning the system, it may typically be preferred to not spray, because the liquid concentration may vary during the flushing process. Recirculation while spraying may be desired to promote uniform mixing of the fluid in the product tank 110 and throughout the liquid distribution system 300. Recirculation and spraying can be selected independently, for example, by controlling system pressure and valve positions. In some embodiments, valves may control flow to individual nozzles 204.

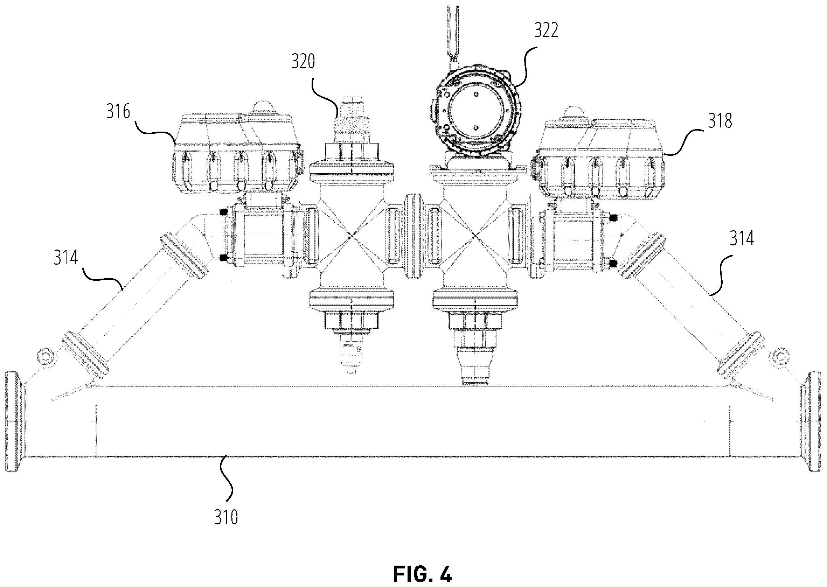

The liquid distribution system 300 shown in FIG. 3 includes a bypass line 314 connected at an upstream connection and a downstream connection to the recirculation line 310. The bypass line 314 is shown in more detail in FIG. 4. The bypass line 314 has at least one (typically two) bypass valves 316, 318 to enable flow of a portion of the recirculated liquid through the bypass line 314. The bypass line 314 includes one or more sensors 320, 322.

The sensors 320, 322 may be configured to measure fluid flowing past or through them. Various types and numbers of sensors 320, 322 may be used. Two sensors 320, 322 are depicted in FIG. 2, but any number of sensors may be used. Furthermore, though the sensors 320, 322 are shown one downstream of the other, the sensors may be arranged to measure the same volume, or in any other configuration. The sensors 320, 322 may include various additional components, such as a pump configured to provide a calibration standard to the sensors 320, 322 when the bypass valves 316, 318 are closed.

The sensors 320, 322 may include, for example, a water quality sensor such as a pH sensor, a turbidity sensor, a dissolved oxygen sensor, a salinity sensor, an ammonia sensor, a chloride sensor, a nitrate sensor, a sulfite sensor, a total organic carbon sensor, a sensor for detecting any other element or ion, or any other selected sensor. In some embodiments, the sensors 320, 322 may include an instrument capable of detecting multiple water quality properties, such as those available from Eureka Water Probes, of Austin, Texas, under the names Trimeter and Manta+.

Typically, the bypass valves 316, 318 are only opened when it is desired to test a portion of the liquid flowing recirculating through the recirculation line 310. Thus, the sensors 320, 322 are not continuously exposed to flowing liquid when the sprayer vehicle 102 is spraying liquid, or even when the liquid is being recirculated for flushing or cleaning. The bypass valves 316, 318 can be opened near the end of a flushing or cleaning process, or can be opened selectively during a spraying operation (*e*.*g*., for a quality control check, or when a spray parameter is changed).

When a spraying operation is complete, it is beneficial to clean the product tank 110 and the plumbing to avoid damaging the liquid distribution system 300 (*e*.*g*., by scale, rust, corrosion, *etc.*) Furthermore, the next material to be sprayed may be different than the current material, and so the current material is cleaned out to avoid contamination of the next material. One way of cleaning the liquid distribution system 300 is to drain the liquid from the product tank 110, then refill the product tank 110 at least partially with clean water. The clean water is flushed through the pump 306, past the nozzles 204, and through the recirculation line 310. The flushed water either goes back into the product tank 110, or is purged from the system to a rinse collection container 324 or the ground. The rinse collection container 324 may be a stationary holding tank, but may alternatively be a tank carried by the sprayer vehicle 102 itself if in-field cleaning is required. A valve 326 controls whether the flushed water is recirculated or purged. In some embodiments, the valve 326 may alternatively turn off flow through the recirculation line 310 altogether, which may be useful when it is desired to spray without recirculation.

To test whether the liquid distribution system 300 is adequately clean, the bypass valves 316, 318 are opened, which allows at least a portion of the flushed water to pass the 320 and the sensors 320, 322. The sensors 320, 322 can then test one or more water quality properties of the flushed water and report the result to an operator of the sprayer vehicle 102.

In one embodiment, a small amount of clean water is added to the product tank 110, and that water is continuously recycled through the liquid distribution system 300 for a period of time to rinse the plumbing lines. The liquid is then purged to the rinse collection container 324. The process is repeated with one or more additional amounts of clean water. It is common to flush with clean water three times, but any number of rinse cycles may be used. The concentration of contaminants decreases step-wise with each rinse cycle. If the rinse water is tested, an operator or a computer (*e*.*g*., within the operator cab 106) can determine an appropriate number of rinse cycles to perform to attain a selected contaminant concentration.

In another embodiment, an amount of clean water is added to the product tank 110, and that water is pushed through the system and to the rinse collection container 324, not returning to the product tank 110. The concentration of contaminants decreases continually as the rinse water passes through the plumbing lines. If the rinse water is tested, an operator or a computer (*e*.*g*., within the operator cab 106) can determine an appropriate amount of rinse water to use to attain a selected contaminant concentration.

In some embodiments, a computer may compare the current value of a water quality property to a threshold value of the water quality property (*e*.*g*., a previously measured water quality property of fresh water), and this information may be used by the operator to determine whether the liquid distribution system 300 is clean enough to switch to distributing a different material.

For example, after the sprayer vehicle 102 has been used to apply one material (*e.g.,* an herbicide), and another material is to be applied next (*e.g.,* a fungicide), the product tank 110 may be at least partially drained of the liquid therein. Removing all of the liquid from the product tank 110 and the liquid distribution system 300 is typically not feasible in operational settings, and therefore a rinse cycle is used to protect crops to be subsequently treated (which crops could be damaged by the material applied previously). Thus, fresh water is provided to the product tank 110 and circulated within the liquid distribution system 300 to form a rinsate (*i*.*e*., a mixture of the fresh water with the residual liquid from the prior spraying operation).

After a period of time, the rinsate may have a substantially uniform concentration of the material previously applied (*e*.*g*., the herbicide, which is at this time, a contaminant). The bypass valves 316, 318 are opened, and the sensors 320, 322 can then measure a water quality property of the rinsate.

The operator or a computer may use the information about the water quality property to determine whether additional rinsing is needed (*e*.*g*., with a fresh batch of fresh water) by comparing the water quality property of the rinsate with a standard (*e*.*g*., a concentration of a particular contaminant in fresh water, or a preselected material concentration).

If the difference between the water quality property of the rinsate and the water quality property of fresh water is greater than a preselected threshold (*i*.*e*., the rinsate is not "clean" enough for the next operation), the rinsate is removed from the product tank 110, and additional fresh water is provided to the product tank 110. The process can be repeated as many times as necessary until a desired difference between the rinsate and fresh water is observed. The rinsate (or second rinsate, third rinsate, *etc.*) is drained from the product tank 110, and the product tank 110 can be filled with the next liquid to be applied (*e*.*g*., the fungicide, and optionally including water to yield a preselected concentration), or the product tank 110 can be kept empty until its next use. The rinsate can be drained by spraying it out the nozzles 204, thus cleaning the nozzles 204 themselves. Alternatively, the rinsate may be withdrawn from the liquid distribution system 300 to the rinse collection container 324, or may be flushed through a drain valve on the boom 112 or elsewhere in the liquid distribution system 300.

The product tank and plumbing lines of conventional sprayer vehicles are typically flushed with fresh water and/or a cleaning solution (*e.g.*, a surfactant, a solvent, *etc.*) according to prescribed procedures or protocols. For example, an equipment or chemical manufacturer may recommend rinsing equipment once with a specified cleaning solution, and twice with fresh water, after applying a certain product. However, there is typically no way to know in the field whether any particular cleaning protocol has been effective without collecting and sending rinsate to a lab for analysis, which is not typically done because of time and expense.

Furthermore, differences in the properties of rinse water can affect how well the product tank and plumbing lines are cleaned. Therefore, having a way to measure water quality properties with the sensors 320, 322 can help operators ensure that their equipment is clean, while avoiding excessive cleaning (which cleaning takes time and produces waste water).

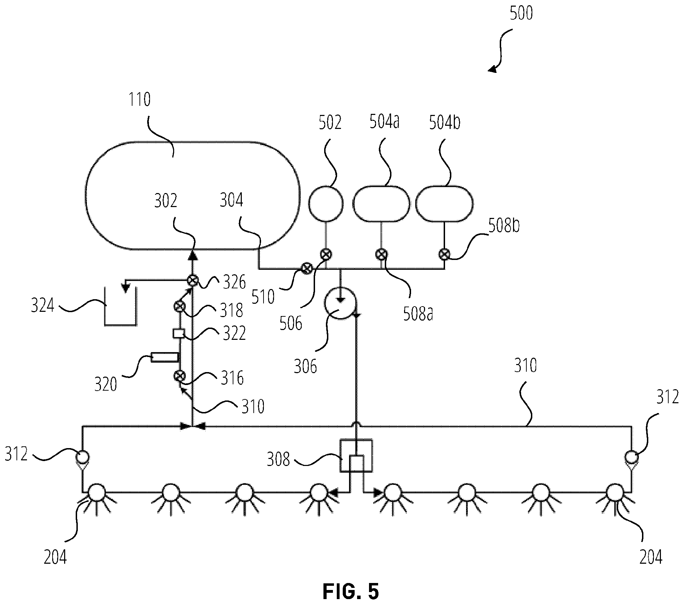

FIG. 5 is a simplified schematic view of another liquid distribution system 500, such as the liquid distribution system 202 carried by the sprayer vehicle 102. The liquid distribution system 500 is similar to the liquid distribution system 300 shown in FIG. 3, but also includes a clean rinse tank 502 and/or chemical tanks 504a, 504b. The liquid distribution system 500 may include valves 506, 508a, 508b for controlling flow from the clean rinse tank 502 and chemical tanks 504a, 504b. A valve 510 may control flow from the product tank 110. The valves 506, 508a, 508b, 510 may be controlled by a central computer, such as in the operator cab 106 of the sprayer vehicle 102. The valves 506, 508a, 508b, 510 may be controlled to deliver selected quantities or concentrations of various products to the field.

For example, the product tank 110 may contain a first product diluted with water to a selected concentration. The first chemical tank 504a may contain a second product in concentrated form, and the second chemical tank 504b may contain a third product in concentrated form. The valves 508a, 508b, 510 may vary the relative concentrations of the first, second, and third products delivered to the nozzles 204 and sprayed on the field. The valve 326 may be kept closed while spraying to maintain the quality of the product in the product tank 110. When the time comes to clean the liquid distribution system 500, the valves 508a, 508b, 510 are closed, and the valve 506 is opened to selectively provide fresh water through the pump 306, flow splitter 308, and recirculation line 310. The fresh water is typically expelled to the rinse collection container 324 or onto the ground, but may also be recirculated through the product tank 110 as described above.

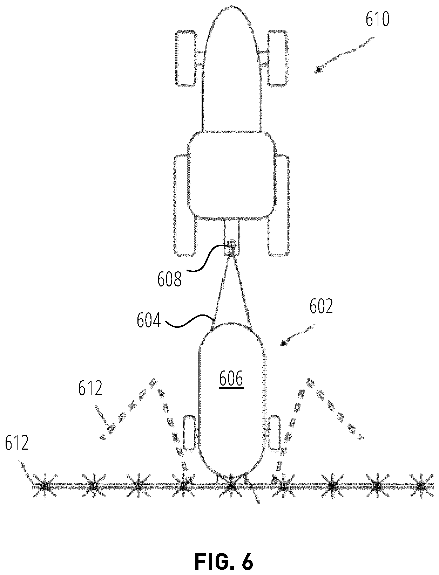

FIG. 6 shows another sprayer vehicle 602 that may be used to deliver chemicals to agricultural crops in a field. The sprayer vehicle 602 is a pull-type sprayer including a towed chassis 604 carrying a product tank 606. The sprayer vehicle 602 has a hitch 608 configured to couple the chassis 604 to a tractor 610. The tractor 610 may therefore pull the sprayer vehicle 602 through the field, and the operator of the tractor 610 may also operate the sprayer vehicle 602 via a control system in the cab of the tractor 610. The boom 112 may have arms that fold for road transport (partially folded boom arms indicated by dashed lines in FIG. 6). The sprayer vehicle 602 may also include a liquid distribution system 300, 500 as shown in FIG. 3 and FIG. 5 and described above.

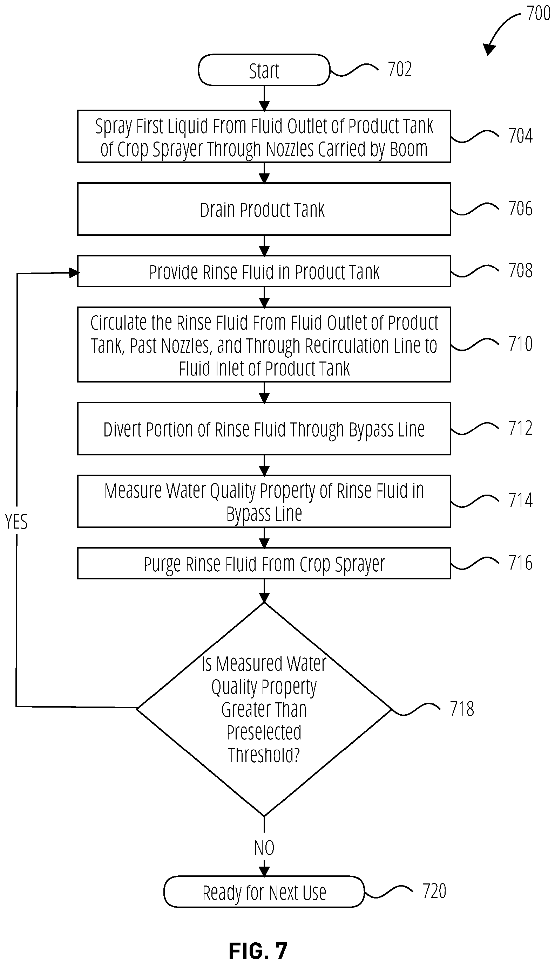

FIG. 7 is a simplified flow chart illustrating a method 700 of operating a sprayer vehicle, such as the sprayer vehicle 102. The method starts at 702. In block 704, a first liquid is sprayed from a fluid outlet of a product tank of the sprayer vehicle through a plurality of nozzles carried by a boom.

In block 706, the product tank is drained of the first liquid. The product tank may be drained by spraying the product out the nozzles, draining into a collection container, or any other method.

In block 708, a rinse fluid is provided in the product tank. The rinse fluid is typically fresh water, but may optionally include a surfactant, a solvent, or other material to facilitate cleaning.

In block 710, the rinse fluid circulates from the fluid outlet of the product tank, past the plurality of nozzles, and through a recirculation line to a fluid inlet of the product tank.

In block 712, at least a portion of the rinse fluid is diverted through a bypass line.

In block 714, a water quality property of the rinse fluid is measured in the bypass line. The water quality property may include, for example, pH, turbidity, dissolved oxygen, salinity, ammonia concentration, chloride concentration, nitrate concentration, sulfite concentration, total organic carbon, a concentration of an element, and/or a concentration of any ion.

In block 716, the rinse fluid is purged from the sprayer vehicle.

As shown at 718, if the measured water quality property is greater than a preselected threshold, additional rinse fluid is provided to the product tank. Blocks 708 through 716 may be repeated as many times as desired. If the measured water quality property is less than the preselected threshold, the sprayer vehicle is ready for its next use (*e*.*g*., to spray a second liquid), storage, *etc.*

Though depicted as a flow chart, actions in FIG. 7 may be performed concurrently, and in some embodiments, some actions may be omitted.

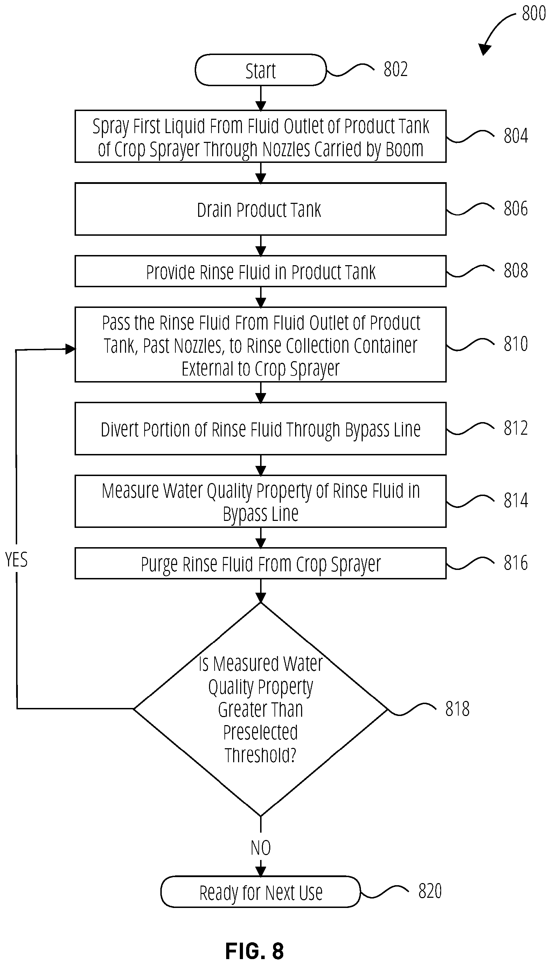

FIG. 8 is a simplified flow chart illustrating another method 800 of operating a sprayer vehicle, such as the sprayer vehicle 102. The method starts at 802. In block 804, a first liquid is sprayed from a fluid outlet of a product tank of the sprayer vehicle through a plurality of nozzles carried by a boom.

In block 806, the product tank is drained of the first liquid. The product tank may be drained by spraying the product out the nozzles, draining into a collection container, or any other method.

In block 808, a rinse fluid is provided in the product tank. The rinse fluid is typically fresh water, but may optionally include a surfactant, a solvent, or other material to facilitate cleaning.

In block 810, the rinse fluid passes from the fluid outlet of the product tank, past the plurality of nozzles, and to a rinse collection container external to the sprayer vehicle.

In block 812, at least a portion of the rinse fluid is diverted through a bypass line.

In block 814, a water quality property of the rinse fluid is measured in the bypass line. The water quality property may include, for example, pH, turbidity, dissolved oxygen, salinity, ammonia concentration, chloride concentration, nitrate concentration, sulfite concentration, total organic carbon, a concentration of an element, and/or a concentration of any ion.

In block 816, the rinse fluid is purged from the sprayer vehicle.

As shown at 818, if the measured water quality property is greater than a preselected threshold, the rinse fluid continues to flow. Blocks 810 through 816 may be repeated until a selected water quality property threshold is met. If the measured water quality property is less than the preselected threshold, the sprayer vehicle is ready for its next use (*e.g.*, to spray a second liquid), storage, *etc.*

Though depicted as a flow chart, actions in FIG. 8 may be performed concurrently, and in some embodiments, some actions may be omitted.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure as hereinafter claimed, including legal equivalents thereof. In addition, features from one embodiment may be combined with features of another embodiment while still being encompassed within the scope as contemplated by the inventors. Further, embodiments of the disclosure have utility with different and various vehicle types and configurations.

## Claims

1. A liquid distribution system for a sprayer vehicle, comprising:
a product tank configured to contain a liquid, the product tank having a fluid inlet and a fluid outlet;
a pump in fluid communication with the product tank;
a plurality of nozzles carried by a boom;
a recirculation line connecting the fluid outlet, the pump, at least some of the plurality of nozzles, and the fluid inlet;
a bypass line connected at an upstream connection and a downstream connection to the recirculation line; and
at least one water quality sensor configured to detect a water quality property of liquid flowing through the bypass line.

2. The liquid distribution system of claim 1, further comprising at least one valve configured to control flow through the bypass line.

3. The liquid distribution system of claim 1 or 2, wherein the at least one water quality sensor comprises at least one sensor selected from the group consisting of a pH sensor, a turbidity sensor, a dissolved oxygen sensor, a salinity sensor, an ammonia sensor, a chlorine sensor, a nitrate sensor, a sulfite sensor, and a total organic carbon sensor.

4. The liquid distribution system of any one of claims 1 to 3, wherein the at least one water quality sensor is configured to report the water quality property to an operator of the sprayer vehicle.

5. The liquid distribution system of any one of claims 1 to 4, further comprising a rinse tank configured to contain rinse water separate from the liquid in the product tank and to selectively provide rinse water to the recirculation line.

6. A sprayer vehicle comprising the liquid distribution system of any one of claims 1 to 5.

7. The sprayer vehicle of claim 6, further comprising a self-propelled chassis carrying the liquid distribution system.

8. The sprayer vehicle of claim 6, further comprising a towed chassis carrying the liquid distribution system.

9. A method of operating a sprayer vehicle, the method comprising:
spraying a first liquid from a fluid outlet of a product tank of the sprayer vehicle through a plurality of nozzles carried by a boom;
providing a rinse fluid in the product tank;
passing the rinse fluid from the fluid outlet of the product tank past the plurality of nozzles and through a recirculation line to a fluid inlet of the product tank or to a rinse collection container external to the sprayer vehicle;
diverting at least a portion of the rinse fluid through a bypass line; and
measuring a water quality property of the rinse fluid in the bypass line.

10. The method of claim 9, further comprising draining the product tank before providing the rinse fluid to the product tank.

11. The method of claim 9 or 10, further comprising purging the rinse fluid from the sprayer vehicle.

12. The method of claim 11, wherein purging the rinse fluid from the sprayer vehicle comprises collecting the rinse fluid in a rinse collection container.

13. The method of any one of claims 9 to 12, wherein measuring the water quality property comprises measuring a water quality property selected from the group consisting of pH, turbidity, dissolved oxygen, salinity, ammonia concentration, chloride concentration, nitrate concentration, sulfite concentration, and total organic carbon.

14. The method of any one of claims 9 to 13, wherein measuring the water quality property comprises measuring a concentration of an element or an ion.

15. The method of any one of claims 9 to 14, further comprising:
if the measured water quality property is greater than a preselected threshold, providing additional rinse fluid to the product tank.
